# EUROPÄISCHE PATENTSCHRIFT

(11) **EP 1 491 226 B1**
(45) Veröffentlichungstag und Bekanntmachung des Hinweises auf die Patenterteilung: **25.01.2006**
(21) Anmeldenummer: 04006156.6
(22) Anmeldetag: 16.03.2004
(51) Int. Cl.: A61M 16/00, A61M 16/10

(54) **Verfahren zur Befeuchtung eines Atemgases sowie Vorrichtung zur Beatmung**
Method for humidification of a respiratory gas and a respiratory device
Méthode pour humidification du gaz respiratoire et dispositif respiratoire

(30) Priorität: 27.06.2003 DE 10328931
(43) Veröffentlichungstag der Anmeldung: 29.12.2004
(73) Patentinhaber: Weinmann Geräte für Medizin GmbH & Co. KG, 22525 Hamburg (DE)
(72) Erfinder: Wedler, Wolfgang, 21147 Hamburg (DE); Feldhahn, Karl-Andreas, 22761 Hamburg (DE)
(74) Vertreter: Klickow, Hans-Henning

(56) Entgegenhaltungen:
- WO-A-95/16484
- WO-A-97/42995
- WO-A-98/12965
- US-A- 3 794 026
- US-A- 4 459 982
- US-A- 5 890 490

## Beschreibung

Die Erfindung betrifft eine Vorrichtung zur Beatmung, die eine Atemgasquelle sowie einen Befeuchter zur Erhöhung des Feuchtigkeitsgehaltes des Atemgases aufweist und bei der der Befeuchter mit einer Steuereinrichtung verbunden ist.

Eine Vorrichtung gemäß dem Oberbegriff des Anspruchs 1 ist aus WO-A-95/16484 bekannt.

Atemluftanfeuchter werden in Verbindung mit CPAP-Geräten, Bilevel-Geräten und Beatmungsgeräten zur Anfeuchtung der vom Gerät an den Patienten abgegebenen Atemluft eingesetzt. Im wesentlichen bestehen solche Anfeuchter aus einem beheizbaren Wassertank oder einem Wasservorratsbehälter mit Teilmengenabgabe an ein Heizelement. Der Luftstrom wird über die Oberfläche des angewärmten Wassers geleitet und nimmt auf diesem Weg Feuchtigkeit und Wärme auf. Die relative Erhöhung der Luftfeuchtigkeit ist hauptsächlich abhängig vom Flow, sowie der Luft- und der Wassertemperatur. Bei einem anderen bekannten Verfahren wird die Anfeuchtung durch Einleitung von vernebeltem oder verdampftem Wasser in den Atemstrom realisiert.

Während der Atmung durch die Nase übernehmen die Schleimhäute der oberen Atemwege des Patienten die physiologisch notwendige Anfeuchtung und Erwärmung der Atemluft. Bei nasaler Applikation von CPAP oder Bilevel bleibt diese Situation weitgehend unverändert. Das Atemminutenvolumen (AMV) wird evtl. geringfügig erhöht. Eine zusätzliche Anfeuchtung ist zunächst einmal aber nicht notwendig.

Während der Inspiration geben die Schleimhäute Feuchtigkeit an die vorbeiströmende Luft ab. Die relative Luftfeuchtigkeit erhöht sich dabei auf dem Weg bis in die Alveolen auf fast 100 %. Die Temperatur der Luft wird auf die Körpertemperatur des Patienten gebracht. Während der Exspiration wird ein Teil der aufgenommenen Feuchtigkeit wieder an die Schleimhäute zurückgegeben. Es stellt sich im physiologischen Fall eine ausgeglichene Feuchtebilanz an den Schleimhäuten ein.

Die Feuchtebilanz der Schleimhäute ändert sich aber dramatisch, sobald unter CPAP oder Bilevel eine Leckage zwischen Nase und Mund entsteht. Eine solche Leckage erhöht den Luftstrom und damit die Menge der von den Schleimhäuten anzufeuchtenden Luft. Eine ähnliche Belastung der Schleimhäute entsteht, wenn nasal inspiriert und oral exspiriert wird. Die Rückführung der Luftfeuchtigkeit an die Schleimhäute ist bei dieser Form der Atmung unterbunden. Beide Probleme werden unter der CPAP- oder Bilevel-Beatmung häufig beobachtet und führen zur Abkühlung und Austrocknung der Schleimhäute mit den bekannten Nebenwirkungen wie Trokkenheit, Schwellung oder Entzündung. Darüber hinaus wächst das Infektionsrisiko.

Bei herkömmlichen Atemluftanfeuchtern wird die Anfeuchtungsleistung vom Patienten gewählt und fest eingestellt. Zur Minderung einer Kondensation in Maske und Schlauch werden Warmluftanfeuchter auch druck- und temperaturkompensiert. Der sich ändernde, aktuelle Bedarf des Patienten wird aber nicht berücksichtigt. Die eingestellte Anfeuchterleistung muß so gewählt werden, daß in Phasen hohen Bedarfs eine ausreichende Anfeuchtung gewährleistet ist. In Phasen geringen Bedarfs ist damit aber die Anfeuchterleistung zu groß. Dies hat neben einem erhöhten Wasserverbrauch auch eine erhöhte Kondensation in Schlauch und Maske zur Folge. Kondensat im Schlauch verschlechtert die Therapie.

Grundsätzlich erfolgt die Geräteeinstellung gemäß dem Stand der Technik derart, daß vom Patienten in der Regel eine zu hohe Anfeuchtung gewählt wird, da lediglich eine zu geringe Befeuchtung zu für den Patienten unangenehmen Auswirkungen führt und eine zu hohe Anfeuchtung für den Patienten zunächst nicht direkt bemerkbar durch Feuchtigkeitskondensation ausgeglichen wird. Die Kondensationseffekte führen jedoch zu einer verminderten Lebensdauer der Geräte, darüber hinaus wird die Ansiedlung von Keimen im Gerätebereich begünstigt.

Weitere Aufgabe der vorliegenden Erfindung ist es, eine Vorrichtung der einleitend genannten Art derart zu konstruieren, daß eine für den Patienten angenehme Befeuchtung unterstützt sowie gleichzeitig eine lange Nutzungszeit des Gerätes erreicht wird.

Diese Aufgabe wird erfindungsgemäß dadurch gelöst, daß die Steuereinrichtung an eine Meßeinrichtung zur Erfassung eines flowabhängigen Signals angeschlossen ist und daß die Steuereinrichtung eine Steuercharakteristik zur Vorgabe der Befeuchtungsintensität in Abhängigkeit von einem aufgrund des gemessenen Signals ermittelten Leckageflow aufweist.

Durch die Erfassung des Leckageflows bzw. eines zum Leckageflow korrespondierenden Parameters sowie die Auswertung dieses Meßergebnisses zur Steuerung der Befeuchtungsintensität ist es möglich, einerseits eine vom Patienten als angenehm empfundene Befeuchtung des Atemgases zu realisieren, andererseits aber eine zu hohe Befeuchtung zu vermeiden, die zu gerätetechnischen Beeinträchtigungen und einer Minderung der Nutzungszeit führt. Statt einer fest eingestellten und von der jeweiligen Benutzungssituation unabhängigen Befeuchtung kann somit adaptiv eine Anpassung an die jeweilige Anwendungssituation derart erfolgen, daß ohne Beeinträchtigung des vom Patienten wahrgenommenen Benutzungskomforts eine verbesserte Gerätelebensdauer unterstützt wird.

Eine typische Anwendung besteht darin, daß meßtechnisch eine Nase-Mund-Leckage erfaßt wird.

Darüber hinaus ist daran gedacht, daß ein oraler Exspirationsflow meßtechnisch erfaßt wird.

Bei einer Ausführungsvariante ist vorgesehen, daß eine patientennahe Flowmessung durchgeführt wird.

Eine andere Ausführungsvariante wird dadurch definiert, daß eine patientenferne Flowmessung durchgeführt wird.

Eine indirekte Flowerfassung kann dadurch erfolgen, daß im Bereich des Beatmungsschlauches eine Druckmessung durchgeführt wird.

Eine Bestimmung des Leckageflows aufgrund eines ermittelten Leckagevolumens kann dadurch erfolgen, daß der Leckageflow in Abhängigkeit von der Beatmungsfrequenz ermittelt wird.

Zu einem ruhigen Steuerungsverhalten trägt es bei, daß der Leckageflow durch eine Differenzbildung zwischen einem Flowmittelwert und einem Spülflowmittelwert berechnet wird.

Bei einer Verwendung von Flowsensoren ist es möglich, daß eine direkte meßtechnische Erfassung des Flows durchgeführt wird.

Eine Anwendung anderer Meßverfahren wird dadurch ermöglicht, daß eine indirekte meßtechnische Erfassung des Flows durchgeführt wird.

In den Zeichnungen sind Ausführungsbeispiele der Erfindung schematisch dargestellt. Es zeigen:
- Fig. 1: Eine perspektivische Darstellung eines Beamtungsgerätes mit Verbindungsschlauch zu einer Beatmungsmaske,
- Fig. 2: einen Flowverlauf bei patientennaher Messung ohne Leckage im Patientenbereich,
- Fig. 3: ein Flowverlauf bei patientennaher Messung mit Leckage im Patientenbereich,
- Fig. 4: ein Verlauf des Spülflows bei CPAP-Beatmung und patientenferner Messung,
- Fig. 5: ein Verlauf des Spülflows bei Bilevel-Beatmung und patientenferner Messung,
- Fig. 6: einen Flowverlauf bei patientenferner Messung ohne Leckage im Bereich des Patienten,
- Fig. 7: ein Flowverlauf bei patientenferner Messung mit Leckage im Patientenbereich,
- Fig. 8: ein Blockschaltbild zur Veranschaulichung der gerätetechnischen Realisierung bei einer patientennahen Flowmessung und
- Fig. 9: ein Blockschaltbild zur Veranschaulichung der gerätetechnischen Realisierung bei einer patientenfernen Flowmessung.

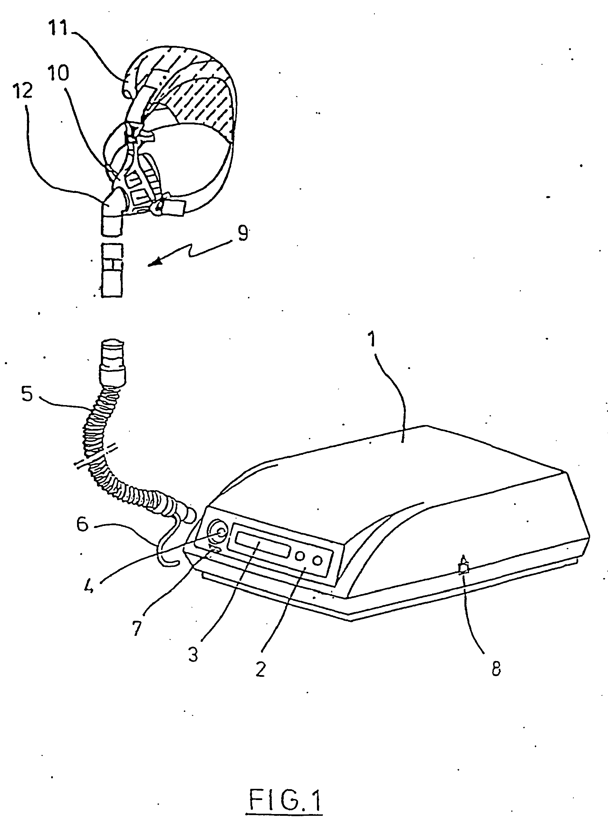

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

Bei einem Einschlauchsystem mit patientennahem Ausatemsystem kann der inspiratorische und exspiratorische Flow meßtechnisch erfaßt werden. Das Ausatemsystem besteht typischerweise aus einem Leckagesystem zur Ausspülung des exspiratorischen Kohlendioxids. Bei einer patientennahen Messung erfolgt die Flowmessung zwischen der Atemmaske und dem Ausatemsystem. Das Leckagevolumen des Patienten je Atemzug errechnet sich aus dem Integral des inspiratorischen Flows abzüglich des Integrals über den exspiratorischen Flow.

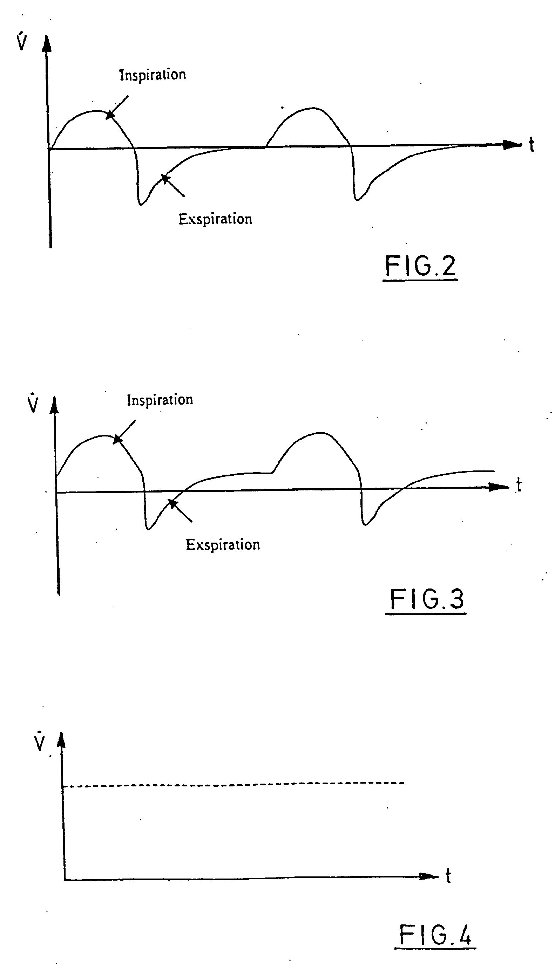

Fig. 2 veranschaulicht einen Flowverlauf bei patientennaher Messung ohne Auftreten einer Leckage im Bereich des Patienten. Fig. 3 zeigt den zugehörigen Flow mit den Änderungen, die sich durch eine Leckage im Bereich des Patienten ergeben.

Bei einer patientenfernen Messung erfolgt die Flowmessung im Beatmungsgerät oder in einer Umgebung des Beatmungsgerätes. Der gesamte Flow wird hierbei als Summe des Patientenflows und des Spülflows ermittelt. Bei einer Kenntnis der Kennlinie des Ausatemsystems kann der Spülflow als Funktion des Druckes im Schlauchsystem separat ermittelt werden.

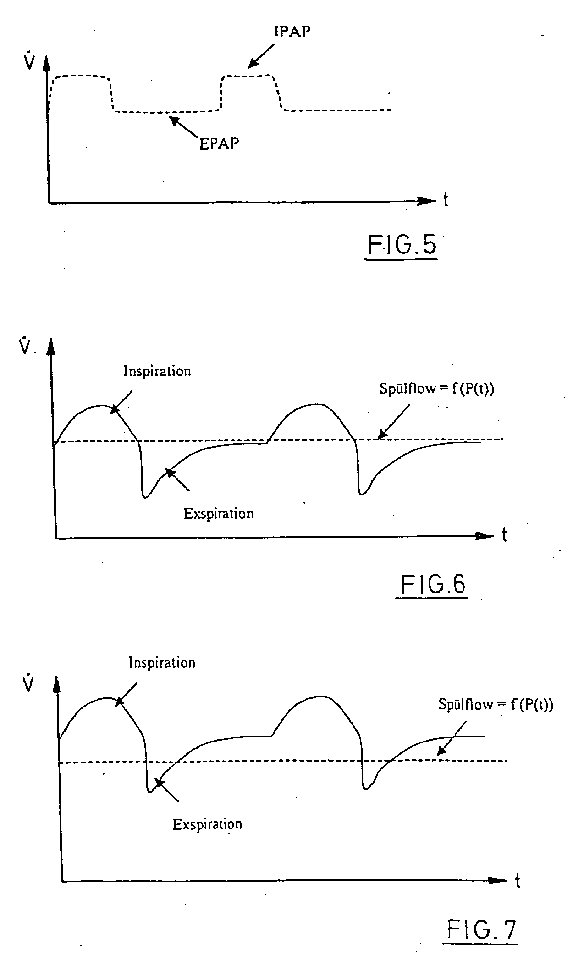

Fig. 4 veranschaulicht für eine patientenferne Messung den Spülflow bei einer CPAP-Beatmung und Fig. 5 veranschaulicht den Spülflow bei einer Bilevel-Beatmung.

Zur Bestimmung des Leckagevolumens des Patienten wird vom Gesamtleckagevolumen das Volumen des Spülflows subtrahiert. Der Leckageflow errechnet sich unter Berücksichtigung der Atemfrequenz als Produkt des Leckagevolumens des Patienten und der Atemfrequenz.

Fig. 6 veranschaulicht für eine patientenferne Messung den Flowverlauf ohne Auftreten einer Leckage im Bereich des Patienten. Fig. 7 veranschaulicht für diese Anwendung den Flowverlauf bei Auftreten einer Leckage im Bereich des Patienten.

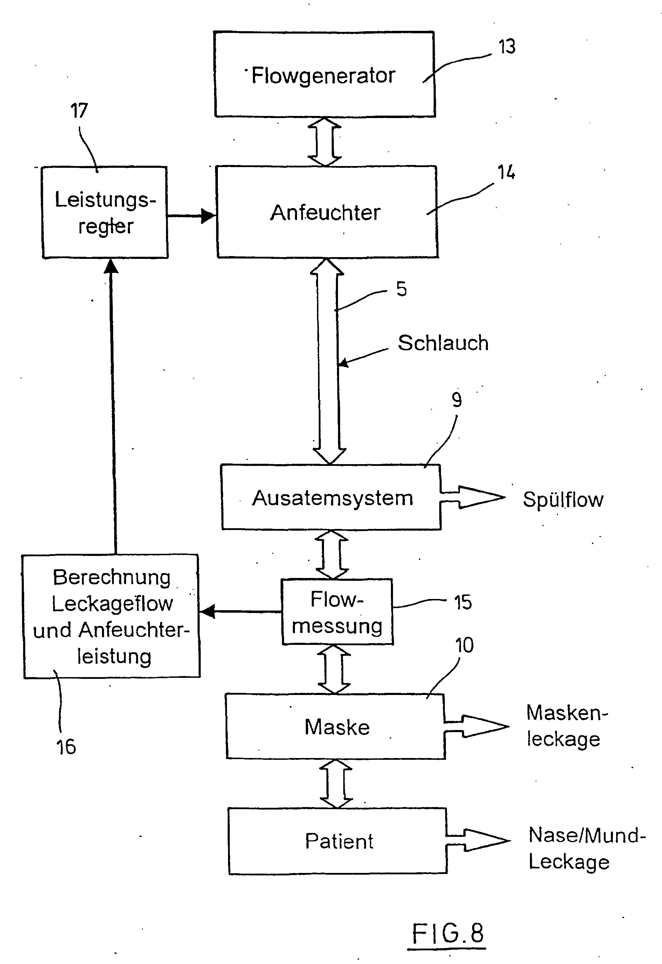

Fig. 8 veranschaulicht den grundsätzlichen Vorrichtungsaufbau sowie den Verfahrensablauf bei einer patientennahen Flowmessung. Ein Flowgenerator (13) wirkt mit einem Anfeuchter (14) zusammen, der angefeuchtete Luft in den Bereich des Beatmungsschlauches (5) leitet. Vom Ausatemsystem (9) wird das Atemgas über eine Atemmaske (10) zu einem Patienten geleitet. Typischerweise zwischen dem Ausatemsystem (9) und der Atemmaske (10) erfolgt eine Flowmessung durch eine Meßeinrichtung (15). Aus dem Ausatemsystem (9) tritt der Spülflow aus. Im Bereich der Maske (10) kann eine Maskenleckage auftreten und im Bereich des Patienten ist mit Leckagen im Nasen- sowie Mundbereich zu rechnen.

Aufgrund der erfolgten Flowmessung erfolgt im Bereich einer Steuereinrichtung (16) eine Berechnung des Leckageflows und der Anfeuchterleistung. Der entsprechende Steuerwert wird einem Leistungsregler (17) für den Anfeuchter (14) zugeführt.

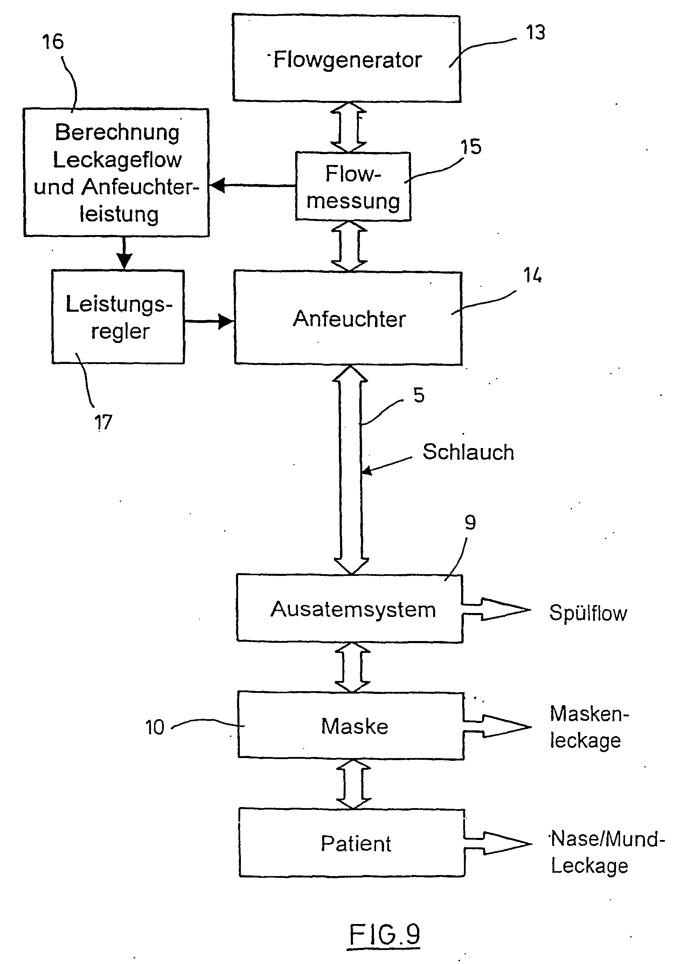

Fig. 9 veranschaulicht den grundsätzlichen Geräteaüfbau sowie den verfahrensablauf für eine patientenferne Flowmessung. Die Fiowmessung erfolgt hierbei typischerweise zwischen dem Flowgenerator (13) und dem Anfeuchter (14). Im übrigen entspricht die Realisierung im wesentlichen der Erläuterung zu Fig. 8.

## Patentansprüche

1. Vorrichtung zur Beatmung, die eine Atemgasquelle sowie einen Befeuchter zur Erhöhung des Feuchtigkeitsgehaltes des Atemgases aufweist und bei dem der Befeuchter mit einer Steuereinrichtung verbunden ist, wobei die Steuereinrichtung (16) an eine Meßeinrichtung (15) zur Erfassung eines flowabhängigen Signals angeschlossen ist, **dadurch gekennzeichnet, daß** die Steuereinrichtung (16) eine Steuercharakteristik zur Vorgabe der Befeuchtungsintensität in Abhängigkeit von einem aufgrund des gemessenen Signals ermittelten Leckageflow aufweist.

2. Vorrichtung nach Anspruch 1, **dadurch gekennzeichnet, daß** die Meßeinrichtung (15) als ein Flowsensor ausgebildet ist.

3. Vorrichtung nach Anspruch 1, **dadurch gekennzeichnet, daß** die Meßeinrichtung (15) als ein Drucksensor ausgebildet ist.

4. Vorrichtung nach einem der Ansprüche 1 bis 3, **dadurch gekennzeichnet, daß** die Meßeinrichtung (15) im Bereich der Atemgasquelle angeordnet ist.

5. Vorrichtung nach einem der Ansprüche 1 bis 3, **dadurch gekennzeichnet, daß** die Meßeinrichtung (15) im Bereich einer Beatmungsmaske (10) angeordnet ist.

6. Vorrichtung nach einem der Ansprüche 1 bis 3, **dadurch gekennzeichnet, daß** die Meßeinrichtung (15) im Bereich eines Beatmungsschlauches (5) angeordnet ist.

7. Vorrichtung nach einem der Ansprüche 1 bis 6, **dadurch gekennzeichnet, daß** die Steuereinrichtung (16) mindestens einen Mittelwertbildner aufweist.

8. Vorrichtung nach einem der Ansprüche 1 bis 7, **dadurch gekennzeichnet, daß** die Atemgasquelle als eine Atemgaspumpe ausgebildet ist.

## Claims

1. Respiratory device which comprises a respiratory gas source and a humidifier for increasing the moisture content of the respiratory gas, and in which the humidifier is connected to a control apparatus, wherein the control apparatus (16) is connected to a measuring apparatus (15) for detecting a flow-dependent signal, **characterised in that** the control device (16) has a control characteristic for predetermining the humidification intensity in accordance with a leakage flow established on the basis of the measured signal.

2. Device according to Claim 1, **characterised in that** the measuring apparatus (15) is formed as a flow sensor.

3. Device according to Claim 1, **characterised in that** the measuring apparatus (15) is formed as a pressure sensor.

4. Device according to any one of Claims 1 to 3, **characterised in that** the measuring apparatus (15) is disposed in the region of the respiratory gas source.

5. Device according to any one of Claims 1 to 3, **characterised in that** the measuring apparatus (15) is disposed in the region of a respiratory mask (10).

6. Device according to any one of Claims 1 to 3, **characterised in that** the measuring apparatus (15) is disposed in the region of a respiratory hose (5).

7. Device according to any one of Claims 1 to 6, **characterised in that** the control apparatus (16) comprises at least one averager.

8. Device according to any one of Claims 1 to 7, **characterised in that** the respiratory gas source is formed as a respiratory gas pump.

## Revendications

1. Dispositif respiratoire, qui comprend une source de gaz respiratoire de même qu'un humidificateur pour augmenter la teneur en humidité du gaz respiratoire, et dans lequel l'humidificateur est relié à un dispositif de commande, le dispositif de commande (16) étant connecté à un dispositif de mesure (15) pour produire un signal dépendant du flux, **caractérisé en ce que** le dispositif de commande (16) présente une caractéristique de commande pour ajuster l'intensité de l'humidification en fonction d'un flux de fuite déterminé sur base du signal mesuré.

2. Dispositif selon la revendication 1, **caractérisé en ce que** le dispositif de mesure (15) est réalisé sous la forme d'un détecteur de flux.

3. Dispositif selon la revendication 1, **caractérisé en ce que** le dispositif de mesure (15) est réalisé sous la forme d'un détecteur de pression.

4. Dispositif selon l'une des revendications 1 à 3, **caractérisé en ce que** le dispositif de mesure (15) est disposé dans la région d'une source de gaz respiratoire.

5. Dispositif selon l'une des revendications 1 à 3, **caractérisé en ce que** le dispositif de mesure (15) est disposé dans la région d'un masque respiratoire (10).

6. Dispositif selon l'une des revendications 1 à 3, **caractérisé en ce que** le dispositif de mesure (15) est disposé dans la région d'un tuyau respiratoire (5).

7. Dispositif selon l'une des revendications 1 à 6, **caractérisé en ce que** le dispositif de commande (16) comprend au moins élément formateur d'une valeur moyenne.

8. Dispositif selon l'une des revendications 1 à 7, **caractérisé en ce que** la source de gaz respiratoire est réalisée sous la forme d'une pompe à gaz respiratoire.
